# FASCICULE DE BREVET EUROPEEN

(11) **EP 2 344 547 B1**
(45) Date de publication et mention de la délivrance du brevet: **03.12.2014**
(21) Numéro de dépôt: 09744446.7
(22) Date de dépôt: 06.10.2009
(51) Int. Cl.: C08B 37/08, A61K 31/715, A61K 47/36, A61K 31/721, C08B 37/00, C08B 37/02

(54) **POLYSACCHARIDES COMPORTANT DES GROUPES FONCTIONNELS CARBOXYLES SUBSTITUES PAR UN DERIVE D'ALCOOL HYDROPHOBE**
POLYSACCHARIDE ENTHALTEND CARBOXYL-FUNKTIONNELLE GRUPPEN, DIE MIT EINEM DERIVAT EINES HYDROPHOBEN ALKOHOLS SUBSTITUIERT SIND
POLYSACCHARIDES CONTAINING CARBOXYL FUNCTIONAL GROUPS SUBSTITUTED BY A HYDROPHOBIC ALCOHOL DERIVATIVE

(30) Priorité: 06.10.2008 FR 0805506; 06.10.2008 US 136816 P
(43) Date de publication de la demande: 20.07.2011
(73) Titulaire: Adocia, 69003 Lyon (FR)
(72) Inventeur: SOULA, Rémi, 69001 Lyon (FR); SOULA, Olivier, 69330 Meyzieu (FR); SOULA, Gérard, 69330 Meyzieu (FR); CHARVET, Richard, 69140 Rillieux-la-Pape (FR)
(74) Mandataire: Tripoz, Inès
(86) Numéro de dépôt international: PCT/IB2009/007054
(87) Numéro de publication internationale: WO 2010/041119

(56) Documents cités:
- EP-A- 0 609 968
- EP-A- 0 831 143
- EP-A- 1 222 926
- WO-A-2008/038111
- DE-A1- 4 136 324
- US-A1- 2002 143 160
- MA ET AL: "Evaluation of blood circulation of polysaccharide surface-decorated PLA nanoparticles" CARBOHYDRATE POLYMERS, APPLIED SCIENCE PUBLISHERS, LTD. BARKING, GB, vol. 72, no. 1, 11 janvier 2008 (2008-01-11), pages 75-81, XP022418394 ISSN: 0144-8617

## Description

La présente invention concerne de nouveaux polymères biocompatibles à base de polysaccharides comportant des groupes fonctionnels carboxyles pouvant être utiles, notamment pour l'administration de principe(s) actif(s) (PA) aux hommes ou aux animaux dans un but thérapeutique et/ou prophylactique.

Les alcools hydrophobes présentent un intérêt dans la formulation de principes actifs pharmaceutiques, notamment, en raison de leur biocompatibilité et de leur caractère hydrophobe permettant de moduler l'hydrophobicité des polymères sur lesquels ils peuvent être greffés.

Leur biocompatibilité est excellente dans la mesure où ils jouent un rôle dans de nombreux processus biochimiques et sont présents sous forme estérifiée dans la plupart des tissus.

Cependant, il est connu de l'homme de l'art qu'il est difficile de greffer un alcool sur un polysaccharide comportant des groupes fonctionnels carboxyles puisqu'il est difficile d'être sélectif entre les fonctions hydroxyles du polysaccharide et la fonction hydroxyle de l'alcool hydrophobe. Au moment du greffage, les alcools du polymère peuvent entrer en compétition avec l'alcool du greffon si l'on ne souhaite pas avoir recours à des techniques de protection déprotection des alcools du polymère et cette réaction secondaire conduit à la réticulation des chaînes de polymère. Ainsi, des alcools hydrophobes d'intérêt tel que le cholésterol n'ont pu être greffés à ce jour sur des polysaccharides comportant des groupes fonctionnels carboxyles.

Dellacherie et al. ont mis au point des esters de polysaccharides, soit d'alginates, de hyaluronates (Pelletier, S. et al., Carbohydr.Polym. 2000, 43, 343-349.) ou de galacturonanes (Dellacherie, Edith et al., Langmuir 2001, 17, 1384-1391.) par une méthode de synthèse employant des alpha halogénures d'alkyle, bromododécane et bromooctadécane. La synthèse des esters consiste à substituer les halogénures par les carboxylates de tetrabutylammonium. Cette méthode permet d'accéder à des esters d'alcools hydrophobes mais elle est limitée aux dérivés halogénés alkyliques pouvant subir une substitution nucléophile. Elle ne peut donc pas être mise en oeuvre pour greffer des alcools hydrophobes tels que le cholestérol. De plus, ces dérivés halogénés présentent des risques de toxicité et ne sont donc pas d'une mise en oeuvre sûre pour le développement d'un produit pharmaceutique.

D'autres chercheurs ont contourné cette difficulté en greffant des acides hydrophobes à la place des alcools hydrophobes. Nichifor et al., par exemple, ont employé l'acide cholique, un dérivé stéroïdien, pour le greffer directement sur les alcools du dextrane (Nichifor, Marieta et al., Eur.Polym.J. 1999, 35, 2125-2129.). Cette méthode contourne le problème du cholésterol en employant un dérivé présentant un acide carboxylique capable de réagir avec les alcools du polysaccharide. Cependant, l'acide cholique n'est pas approuvé par la FDA pour des injections contrairement au cholestérol et cette stratégie ne peut pas être mise en oeuvre avec des polysaccharides comportant des groupes fonctionnels carboxyles.

D'autres chercheurs ont employé des polysaccharides non anioniques afin de pouvoir greffer des alcools hydrophobes. Akiyoshi et al., par exemple, ont converti le cholestérol, nucléophile, en un dérivé électrophile (Biomacromolecules 2007, 8, 2366-2373). Ce dérivé électrophile du cholestérol a pu être greffé sur les fonctions alcools du pullulane ou du mannane, polysaccharides neutres. Cette stratégie ne peut également pas être mise en oeuvre avec des polysaccharides comportant des groupes fonctionnels carboxyles.

Une revue récente des polymères fonctionnels à base de dextrane (Heinze, Thomas et al., Adv Polym Sci 2006, 205, 199-291.) fait état de modifications par des acides hydrophobes entre autres mais ne fait pas état de dextrane fonctionnalisé par des alcools hydrophobes. Le document WO 2008/038111 A1 décrit des dextranes fonctionalisés par des amino-acides hydrophobes destinés à être utilisés dans des compositions pharmaceutiques pour l'administration de principes actifs.

La présente invention concerne de nouveaux dérivés de polysaccharides amphiphiles comportant des groupes fonctionnels carboxyles en partie substitués par au moins un dérivé d'alcool hydrophobe. Ces nouveaux dérivés de polysaccharides comportant des groupes fonctionnels carboxyles ont une bonne biocompatibilité et leur hydrophobicité est facilement modulable sans altérer la biocompatibilité.

Elle concerne également une méthode de synthèse permettant de résoudre les problèmes de synthèse supra cités. Cette méthode a permis d'obtenir des polysaccharides comportant des groupes fonctionnels carboxyles en partie substitués par des alcools hydrophobes dont par exemple le cholestérol.

L'invention concerne donc des polysaccharides comportant des groupes fonctionnels carboxyles dont un au moins est substitué par un dérivé d'alcool hydrophobe, noté Ah :
- ledit alcool hydrophobe (Ah) étant greffé ou lié au polysaccharide anionique par un bras de couplage R, ledit bras de couplage étant lié au polysaccharide anionique par une fonction F ladite fonction F résultant du couplage entre la fonction amine du bras de liaison R et une fonction carboxyle du polysaccharide anionique, et ledit bras de couplage étant lié à l'alcool hydrophobe par une fonction G résultant du couplage entre une fonction carboxyle, isocyanate, thioacide ou alcool du bras de couplage et une fonction de l'alcool hydrophobe, les fonctions carboxyles du polysaccharide anionique non substituées étant sous forme de carboxylate de cation, alcalin de préférence comme Na⁺ ou K⁺.
   - F étant une fonction amide,
   - G étant soit une fonction ester, thioester, carbonate, carbamate,
   - R étant une chaîne comprenant entre 1 et 18 carbones, éventuellement branchée et/ou insaturée, éventuellement comprenant un ou plusieurs hétéroatomes, tels que O, N ou/et S, et ayant au moins une fonction acide,
- Ah étant un reste d'un alcool hydrophobe, produit du couplage entre la fonction hydroxyle de l'alcool hydrophobe et au moins une fonction électrophile portée par le groupement R,
- ledit polysaccharide comportant des groupes fonctionnels carboxyles étant amphiphile à pH neutre.

Dans un mode de réalisation, G est une fonction ester.

Selon l'invention, le polysaccharide comportant des groupes fonctionnels carboxyles en partie substitués par des alcools hydrophobes est choisi parmi les polysaccharides comportant des groupes fonctionnels carboxyles de formule générale I : 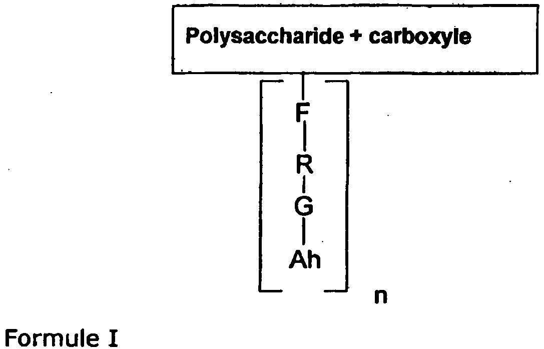
- dans laquelle; n représente la fraction molaire des fonctions carboxyles du polysaccharide substituées par F-R-G-Ah et est compris entre 0,01 et 0,7,
- F, R, G et Ah répondant aux définitions données ci-dessus, et lorsque la fonction carboxyle du polysaccharide n'est pas substituée par F-R-G-Ah, alors le ou les groupes fonctionnels carboxyles du polysaccharide sont des carboxylates de cation, alcalin de préférence comme Na⁺ ou K⁺.

Dans un mode de réalisation, les polysaccharides comportant des groupes fonctionnels carboxyles sont des polysaccharides naturellement porteurs de groupes fonctionnels carboxyles et sont choisis dans le groupe constitué par l'alginate, le hyaluronane, le galacturonane.

Dans un mode de réalisation, les polysaccharides comportant des groupes fonctionnels carboxyles sont des polysaccharides synthétiques obtenus à partir de polysaccharides comportant naturellement des groupes fonctionnels carboxyles ou à partir de polysaccharides neutres, sur lesquels au moins 15 groupes fonctionnels carboxyles pour 100 unités saccharidiques ont été greffées, de formule générale II. 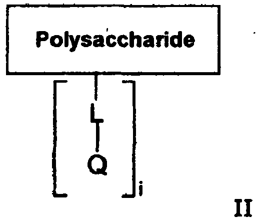
- les polysaccharides naturels étant choisis dans le groupe des polysaccharides constitués en majorité de monomères glycosidiques liés par des liaisons glycosidiques de type (1,6) et/ou (1,4) et/ou (1,3) et/ou (1,2),
- L étant une liaison résultant du couplage entre le bras de liaison Q et une fonction -OH du polysaccharide et étant soit une fonction ester, thionoester, carbonate, carbamate ou éther,
- i représente la fraction molaire des substituants L-Q par unité saccharidique du polysaccharide
- Q étant une chaîne comprenant entre 1 et 18 carbones, éventuellement branchée et/ou insaturée comprenant un ou plusieurs hétéroatomes, tels que O, N ou/et S, et comportant au moins un groupe fonctionnel carboxyle, - CO₂H.

Dans un mode de réalisation, n est compris entre 0,05 et 0,5.

Dans un mode de réalisation le polysaccharide est constitué en majorité de monomères glycosidiques liés par des liaisons glycosidiques de type (1,6).

Dans un mode de réalisation, le polysaccharide constitué en majorité de monomères glycosidiques liés par des liaisons glycosidiques de type (1,6) est le dextrane.

Dans un mode de réalisation le polysaccharide est constitué en majorité de monomères glycosidiques liés par des liaisons glycosidiques de type (1,4).

Dans un mode de réalisation, le polysaccharide constitué en majorité de monomères glycosidiques liés par des liaisons glycosidiques de type (1,4) est choisi dans le groupe constitué par le pullulane, l'alginate, le hyaluronane, le xylane, le galacturonane ou une cellulose soluble dans l'eau.

Dans un mode de réalisation, le polysaccharide est un pullulane.

Dans un mode de réalisation, le polysaccharide est un alginate.

Dans un mode de réalisation, le polysaccharide est un hyaluronane.

Dans un mode de réalisation, le polysaccharide est un xylane.

Dans un mode de réalisation, le polysaccharide est un galacturonane.

Dans un mode de réalisation, le polysaccharide est une cellulose soluble dans l'eau.

Dans un mode de réalisation le polysaccharide est constitué en majorité de monomères glycosidiques liés par des liaisons glycosidiques de type (1,3).

Dans un mode de réalisation, le polysaccharide constitué en majorité de monomères glycosidiques liés par des liaisons glycosidiques de type (1,3) est un curdlane.

Dans un mode de réalisation le polysaccharide est constitué en majorité de monomères glycosidiques liés par des liaisons glycosidiques de type (1,2).

Dans un mode de réalisation, le polysaccharide constitué en majorité de monomères glycosidiques liés par des liaisons glycosidiques de type (1,2) est une inuline.

Dans un mode de réalisation le polysaccharide est constitué en majorité de monomères glycosidiques liés par des liaisons glycosidiques de type (1,4) et (1,3)

Dans un mode de réalisation, le polysaccharide constitué en majorité de monomères glycosidiques liés par des liaisons glycosidiques de type (1,4) et (1,3) est un glucane.

Dans un mode de réalisation le polysaccharide est constitué en majorité de monomères glycosidiques liés par des liaisons glycosidiques de type (1,4), et (1,3) et (1,2).

Dans un mode de réalisation le polysaccharide constitué en majorité de monomères glycosidiques liés par des liaisons glycosidiques de type (1,4), et (1,3) et (1,2) est le mannane.

Dans un mode de réalisation, le polysaccharide selon l'invention est caractérisé en ce que le groupe Q est choisi dans les groupes suivants :

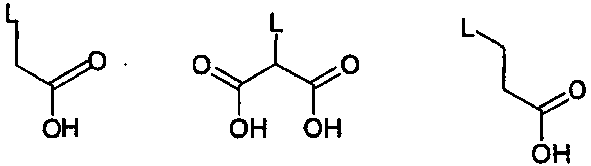

Dans un mode de réalisation, i est compris entre 0,1 et 2.

Dans un mode de réalisation, i est compris entre 0,2 et 1,5.

Dans un mode de réalisation, le groupement R selon l'invention est caractérisé en ce qu'il est choisi parmi les acides aminés.

Dans un mode de réalisation, les acides aminés sont choisis parmi les alpha acides aminés.

Dans un mode de réalisation, les alpha acides aminés sont choisis parmi les alpha acides aminés naturels.

Dans un mode de réalisation, les alpha acides aminés naturels sont choisis parmi la leucine, l'alanine, l'iso-leucine, la glycine, la phénylalanine, le tryptophane, la valine.

Dans un mode de réalisation, l'alcool hydrophobe est choisi parmi les alcools gras.

Dans un mode de réalisation, l'alcool hydrophobe est choisi parmi les alcools constitués d'une chaîne alkyle insaturée ou saturée, ramifiée ou non ramifiée, comprenant de 4 à 18 carbones.

Dans un mode de réalisation, l'alcool hydrophobe est choisi parmi les alcools constitués d'une chaîne alkyle insaturée ou saturée, ramifiée ou non ramifiée, comprenant de 6 à 18 carbones.

Dans un mode de réalisation, l'alcool hydrophobe est choisi parmi les alcools constitués d'une chaîne alkyle insaturée ou saturée, ramifiée ou non ramifiée, comprenant de 8 à 16 carbones.

Dans un mode de réalisation, l'alcool hydrophobe est l'octanol.

Dans un mode de réalisation, l'alcool hydrophobe est le 2-éthylbutanol.

Dans un mode de réalisation, l'alcool gras est choisi parmi le méristyl, le cétyl, le stéaryl, le cétéaryl, le butyl, l'oléyl, la lanoline.

Dans un mode de réalisation, l'alcool hydrophobe est choisi parmi les dérivés du cholestérol.

Dans un mode de réalisation, le dérivé du cholestérol est le cholestérol.

Dans un mode de réalisation, l'alcool hydrophobe est choisi parmi les dérivés du menthol.

Dans un mode de réalisation, l'alcool hydrophobe est le menthol sous sa forme racémique.

Dans un mode de réalisation, l'alcool hydrophobe est l'isomère D du menthol.

Dans un mode de réalisation, l'alcool hydrophobe est l'isomère L du menthol.

Dans un mode de réalisation, l'alcool hydrophobe est choisi parmi les tocophérols.

Dans un mode de réalisation, le tocophérol est l'alpha tocophérol.

Dans un mode de réalisation, l'alpha tocophérol est le racémique de l'alpha tocophérol.

Dans un mode de réalisation, le tocophérol est l'isomère D de l'alpha tocophérol.

Dans un mode de réalisation, le tocophérol est l'isomère L de l'alpha tocophérol.

Dans un mode de réalisation, l'alcool hydrophobe est choisi parmi les alcools porteurs de groupe aryle.

Dans un mode de réalisation, l'alcool porteur de groupe aryle est choisi parmi l'alcool benzylique, l'alcool phenéthylique.

Le polysaccharide peut avoir un degré de polymérisation m compris entre 10 et 10000.

Dans un mode de réalisation, il a un degré de polymérisation m compris entre 10 et 1000.

Dans un autre mode de réalisation, il a un degré de polymérisation m compris entre 10 et 500.

L'invention concerne également la synthèse des polysaccharides comportant des groupes fonctionnels carboxyles en partie substitués selon l'invention.

Ladite synthèse comprend une étape d'obtention d'un intermédiaire aminé Ah-G-R-NH₂ ou d'un sel d'ammonium Ah-G-R-NH₃⁺ dont le contre-ion est un anion choisi parmi les halogénures, les sulfates, les sulfonates, les carboxylates, et une étape de greffage de cet intermédiaire aminé sur une fonction carboxyle d'un polysaccharide, R, G et Ah répondant aux définitions données ci-dessus.

Dans un mode de réalisation une étape de fonctionnalisation du polysaccharide par au moins 15 groupes fonctionnels carboxyles pour 100 unités saccharidiques est effectuée par greffage de composés de formule Q-L', L' étant une fonction anhydride, halogénure, acide carboxylique, thioacide ou isocyanate sur au moins 15 fonctions alcool pour 100 unités saccharidiques du polysaccharide, Q et L répondant aux définitions données ci-dessus.

Dans un mode de réalisation, l'intermédiaire aminé de formule Ah-G-R-NH₂ ou Ah-G-R-NH₃⁺ est obtenu par réaction d'un composé de formule G'-R-NH₂, G' étant une fonction acide carboxylique, isocyanate, thioacide, ou alcool avec la fonction alcool de l'alcool hydrophobe, R, G et Ah répondant aux définitions données ci-dessus.

Si nécessaire dans cette étape d'obtention de l'intermédiaire aminé, les techniques de protection, déprotection bien connues de l'homme de l'art en synthèse peptidique sont utilisées.

De préférence, l'étape de greffage de l'intermédiaire aminé sur une fonction acide du polysaccharide est réalisée en milieu organique.

L'invention concerne également l'utilisation des polysaccharides fonctionnalisés selon l'invention pour la préparation de compositions pharmaceutiques telles que décrites précédemment.

L'invention concerne également une composition pharmaceutique comprenant l'un des polysaccharides selon l'invention tel que décrit précédemment et au moins un principe actif.

L'invention concerne également une composition pharmaceutique selon l'invention telle que décrite précédemment caractérisée en ce que le principe actif est choisi dans le groupe constitué par les protéines, les glycoprotéines, les peptides et les molécules thérapeutiques non-peptidiques.

On entend par principe actif un produit sous forme d'entité chimique unique ou sous forme d'une combinaison ayant une activité physiologique. Ledit principe actif peut être exogène c'est à dire qu'il est apporté par la composition selon l'invention. Il peut également être endogène, par exemple les facteurs de croissance qui vont être sécrétés dans une plaie pendant la première phase de cicatrisation et qui pourront être retenus sur ladite plaie par la composition selon l'invention.

Selon les pathologies visées elle est destinée à un traitement local ou systémique.

Dans le cas des libérations locale et systémique, les modes d'administration envisagés sont par voie intraveineuse, sous-cutanée, intradermique, transdermique, intramusculaire, orale, nasale, vaginale, oculaire, buccale et pulmonaire.

Les compositions pharmaceutiques selon l'invention sont soit sous forme liquide, en solution aqueuse, soit sous forme de poudre, d'implant ou de film. Elles comportent en outre les excipients pharmaceutiques classiques bien connus de l'homme de l'art.

En fonction des pathologies et des modes d'administration les compositions pharmaceutiques pourront avantageusement comporter, en outre, des excipients permettant de les formuler sous forme de gel, d'éponge, de solution injectable, de solution buvable et de lyoc.

L'invention concerne également une composition pharmaceutique selon l'invention telle que décrite précédemment, caractérisée en ce qu'elle est administrable sous forme de stent, de film ou « coating » de biomatériaux implantables, d'implant.

### Exemple 1 : Synthèse de dextraneméthylcarboxylate de sodium modifié par le leucinate de cholestérol

Le leucinate de cholestérol, sel d'acide paratoluènesulfonique est obtenu selon le procédé décrit dans le brevet (Kenji, M et al., US4826818).

8 g (soit 148 mmol de fonctions hydroxyles) de dextrane de masse molaire moyenne en poids d'environ 40 kg/mol (Fluka) sont solubilisés dans de l'eau à 42 g/L. A cette solution sont ajoutés 15 mL de NaOH 10 N (148 mmol NaOH). Le mélange est porté à 35°C puis 23 g (198 mmol) de chloroacétate de sodium sont ajoutés. La température du milieu réactionnel est portée à 60°C à 0,5°C/min puis maintenue à 60°C pendant 100 minutes. Le milieu réactionnel est dilué avec 200 mL d'eau, neutralisé à l'acide acétique et purifié par ultrafiltration sur membrane PES de 5 kD contre 6 volumes d'eau. La solution finale est dosée par extrait sec pour déterminer la concentration en polymère ; puis dosée par dosage acide/base dans de l'eau/acétone 50 / 50 (V/V) pour déterminer le degré de substitution en méthylcarboxylates.

D'après l'extrait sec : [polymère] = 31,5 mg/g

D'après le dosage acide/base : le degré de substitution des fonctions hydroxyles par des fonctions méthylcarboxylates est de 1,04 par motif saccharidique.

La solution de dextraneméthylcarboxylate de sodium est passée sur une résine Purolite (anionique) pour obtenir le dextraneméthylcarboxylique acide qui est ensuite lyophilisé pendant 18 heures.

8 g de dextraneméthylcarboxylique acide (37 mmol fonction méthylcarboxylique acide) sont solubilisés dans le DMF à 45 g/L puis refroidis à 0°C. 0,73 g de leucinate de cholestérol, sel d'acide paratoluènesulfonique (1 mmol) est mis en suspension dans du DMF à 100 g/L. 0,11 g de triéthylamine (1 mmol) est ensuite ajouté à cette suspension. Une fois la solution de polymère à 0°C, 0,109 g (1 mmol) de NMM et 0,117 g (1 mmol) de EtOCOCl sont ensuite ajoutés. Après 10 min de réaction, la suspension de leucinate de cholestérol est ajoutée. Le milieu est ensuite maintenu à 4°C durant 15 minutes. Le milieu est ensuite chauffé à 30°C. Une fois à 30°C, le milieu est ensuite coulé dans une solution de 3,76 g de NMM (37 mmol) à 5 g/L sous vive agitation. La solution est ultrafiltrée sur membrane PES 10 kD contre 10 volumes de solution NaCl 0.9% puis 5 volumes d'eau. La concentration de la solution de polymère est déterminée par extrait sec. Une fraction de solution est lyophilisée et analysée par RMN dans D₂Opour déterminer le taux de fonctions acides converties en amide de leucinate de cholestérol.

D'après l'extrait sec : [polymère modifié] = 12,9 mg/g

D'après la RMN ¹H : la fraction molaire des acides modifiés par le leucinate de cholesterol par unité saccharidique est de 0,03.

### Exemple 2 : Synthèse de dextranesuccinate de sodium modifié par le leucinate de cholestérol

Le leucinate de cholestérol, sel d'acide paratoluènesulfonique est obtenu selon le procédé décrit dans le brevet (Kenji, M et al., US4826818).

Le dextranesuccinate de sodium est obtenu à partir du dextrane 40 selon la méthode décrite dans l'article de Sanchez-Chaves et al. (Sanchez-Chaves, Manuel et al., Polymer 1998, 39 (13), 2751-2757.) Le taux de fonctions acides par unité glycosidique (i) est de 1,46 d'après la RMN ¹H dans D₂O/NaOD.

La solution de dextranesuccinate de sodium est passée sur une résine Purolite (anionique) pour obtenir le dextranesuccinique acide qui est ensuite lyophilisé pendant 18 heures.

7,1 g de dextranesuccinique acide (23 mmol) sont solubilisés dans le DMF à 44 g/L. La solution est refroidie à 0°C. 0,77 g de leucinate de cholestérol, sel d'acide paratoluènesulfonique (1 mmol) est mis en suspension dans du DMF à 100 g/L. 0,12 g de triéthylamine (TEA) (1 mmol) est ensuite ajouté à cette suspension. Une fois la solution de polymère à 0°C, 0,116 g (1 mmol) de NMM et 0,124 g (1 mmol) de EtOCOCl sont ensuite ajoutés. Après 10 min de réaction, la suspension de leucinate de cholestérol est ajoutée. Le milieu est ensuite maintenu à 4°C durant 15 minutes. Le milieu est ensuite chauffé à 30°C. Une fois à 30°C, le milieu est ensuite coulé dans une solution de 3,39 g de NMM (33 mmol) à 5 g/L sous vive agitation. La solution est ultrafiltrée sur membrane PES 10 kD contre 10 volumes de solution NaCl 0,9% puis 5 volumes d'eau. La concentration de la solution de polymère est déterminée par extrait sec. Une fraction de solution est lyophilisée et analysée par RMN ¹H dans D₂O pour déterminer le taux de fonctions acides converties en amide de leucinate de cholestérol.

D'après l'extrait sec : [polymère modifié] = 17,5 mg/g

D'après la RMN ¹H : la fraction molaire des acides modifiés par le leucinate de cholestérol par unité saccharidique est de 0,05.

### Exemple 3 : Synthèse de pullulanesucciniquecarboxylate de sodium modifié par le leucinate de cholestérol

Le leucinate de cholestérol, sel d'acide paratoluènesulfonique est obtenu selon le procédé décrit dans le brevet (Kenji, M et al., US4826818).

10 g de pullulane de masse molaire moyenne en poids d'environ 100 kg/mol (Fluka) est solubilisé dans le DMSO à une concentration de 400 mg/g à 60°C. Cette solution est équilibrée à 40°C puis deux solutions de DMF contenant 9,27 g d'anhydride succinique (371 g/L) et 9,37 g de NMM (375 g/L) sont ajoutées à la solution de pullulane. Le temps de réaction est de 240 min à partir de l'ajout de la solution de NMM. La solution ainsi obtenue est diluée dans 1 L d'eau et ultrafiltrée sur membrane en PES de 10 kD contre de une solution de chlorure de sodium à 0,9% puis contre de l'eau bidistillée. La concentration en pullulanesucciniquecarboxylate de sodium dans la solution finale est déterminée par extrait sec et le produit sec est analysé par RMN ¹H dans D₂O/NaOD pour déterminer le taux de fonctions hydroxyles converties en ester succinique par unité saccharidique.

D'après l'extrait sec : [pullulanesucciniquecarboxylate] = 15,8 mg/g

D'après la RMN ¹H :la fraction molaire des alcools porteurs d'un succinate de sodium par unité saccharidique est de 1,35.

La solution de pullulanesucciniquecarboxylate de sodium est acidifiée sur une résine Purolite (anionique) puis est ensuite lyophilisée pendant 18 heures.

5 g de pullulanesucciniqueacide sont solubilisés dans le DMF à 51 g/L. La solution est refroidie à 0°C. 0,08 g de NMM et 0,08 g de EtOCOCl sont ensuite ajoutés. Après 10 min de réaction, une suspension contenant 0,51 g de leucinate de cholestérol, sel d'acide paratoluènesulfonique (APTS) et 0,08 g de TEA dans 5,1 mL de DMF est ajoutée. Le temps de greffage est de 20 min après l'introduction du dérivé cholestérol. Le milieu est ensuite chauffé à 30°C puis coulé dans une solution aqueuse de NMM (2,09 g à 5 mg/mL). La solution obtenue est diluée en ajoutant 100 mL d'eau puis diafiltrée sur membrane PES de 10 kD contre de une solution de chlorure de sodium à 0.9% puis contre de l'eau bidistillée. La concentration en pullulanesucciniquecarboxylate de sodium modifié par le leucinate de cholestérol dans la solution finale est déterminée par extrait sec et le produit sec est analysé par RMN ¹H dans D₂O/NaOD pour déterminer le taux de fonctions acides converties en amide de leucinate de cholestérol.

D'après l'extrait sec : [polymère modifié] = 2,9 mg/g

D'après la RMN ¹H : la fraction molaire des acides modifiés par le leucinate de cholestérol par unité saccharidique est de 0,04.

### Exemple 4 : Synthèse de pullulanesucciniquecarboxylate de sodium modifié par l'alaninate d'alcool cétylique

L'alaninate d'alcool cétylique est obtenu selon le procédé décrit dans le brevet (Kenji, M et al., US4826818).

Une solution de pullulanesucciniquecarboxylate de sodium obtenue comme décrit à l'exemple 3 est acidifiée sur une résine Purolite (anionique) puis est ensuite lyophilisée pendant 18 heures.

5 g de pullulanesucciniqueacide sont solubilisés dans le DMF à 51 g/L. La solution est refroidie à 0°C. 0,32 g de NMM (3,2 mmol) et 0,32 g de EtOCOCl (3,2 mmol) sont ensuite ajoutés. Après 10 min de réaction, une suspension contenant 1,55 g d'alaninate d'alcool cétylique, sel d'acide paratoluènesulfonique (3,2 mmol) et 0,32 g de TEA (3,2 mmol) dans 20,4 mL de DMF est ajoutée. Le temps de greffage est de 20 min après l'introduction du dérivé alcool cétylique. Le milieu est ensuite chauffé à 30°C puis coulé dans une solution aqueuse de NMM (8,36 g à 5 mg/mL). La solution obtenue est diluée en ajoutant 100 mL d'eau puis diafiltrée sur membrane PES de 10 kD contre une solution de chlorure de sodium à 0,9% puis contre de l'eau bidistillée. La concentration en pullulanesucciniquecarboxylate de sodium modifié par l'alaninate d'alcool cétylique dans la solution finale est déterminée par extrait sec et le produit sec est analysé par RMN ¹H dans D₂O/NaOD pour déterminer le taux de fonctions acides converties en amide d'alaninate d'alcool cétylique.

D'après l'extrait sec : [polymère modifié] = 5,2 mg/g

D'après la RMN ¹H : la fraction molaire des acides modifiés par l'alaninate d'alcool cétylique par unité saccharidique est de 0,18.

### Exemple 5 : Synthèse de dextraneméthylcarboxylate de sodium modifié par l'alaninate de dodécanol

L'alaninate de dodécanol, sel d'acide paratoluènesulfonique est obtenu selon le procédé décrit dans le brevet (Kenji, M et al., US4826818).

Une solution de dextraneméthylcarboxylate de sodium obtenue comme décrite à l'exemple 1 est passée sur une résine Purolite (anionique) pour obtenir le dextraneméthylcarboxylique acide qui est ensuite lyophilisé pendant 18 heures.

5 g de dextraneméthylcarboxylique acide (23,2 mmol fonction méthylcarboxylique acide) sont solubilisés dans le DMF à 45 g/L puis refroidis à 0°C. 1,99 g d' alaninate de dodécanol, sel d'acide paratoluènesulfonique (4,6 mmol) est mis en suspension dans du DMF à 100 g/L. 0,47 g de triéthylamine (4,6 mmol) est ensuite ajouté à cette suspension. Une fois la solution de polymère à 0°C, 2,35 g (23,2 mmol) de NMM et 2,52 g (23,2 mmol) de EtOCOCl sont ensuite ajoutés. Après 10 min de réaction, la suspension d'alaninate de dodécanol est ajoutée. Le milieu est ensuite maintenu à 4°C durant 15 minutes. Le milieu est ensuite chauffé à 30°C. Une fois à 30°C, une solution d'imidazole (3,2 g dans 9,3 mL d'eau) est ajoutée dans le milieu réactionnel. La solution de polymère est ultrafiltrée sur membrane PES 10 kD contre 10 volumes de solution NaCl 0,9% puis 5 volumes d'eau. La concentration de la solution de polymère est déterminée par extrait sec. Une fraction de solution est lyophilisée et analysée par RMN ¹H dans D₂O pour déterminer le taux de fonctions acides modifiées par l'alaninate de dodécanol.

D'après l'extrait sec : [polymère modifié] = 22 mg/g

D'après la RMN ¹H : la fraction molaire des acides modifiés par l'alaninate de dodécanol par unité saccharidique est de 0,19.

### Exemple 6: Synthèse de dextraneméthylcarboxylate de sodium modifié par le glycinate de L-menthol

Le glycinate de L-menthol, sel d'acide paratoluènesulfonique est obtenu selon le procédé décrit dans le brevet (Kenji, M et al., US4826818).

L'huile obtenue contenant des impuretés, le sel d'amine est neutralisé par un ajout stoechiométrique de soude, et extrait avec du diisopropyle d'éther. La phase organique est alors acidifiée avec une solution d'HCl dans l'éther éthylique et le sel HCl du dérivé mentholé extrait avec de l'eau. Après lyophilisation, le glycinate de L-menthol, sel d'acide chlorhydrique, est obtenu.

Une solution de dextraneméthylcarboxylate de sodium obtenue comme décrite à l'exemple 1 est passée sur une résine Purolite (anionique) pour obtenir le dextraneméthylcarboxylique acide qui est ensuite lyophilisé pendant 18 heures.

12 g de dextraneméthylcarboxylique acide (59,22 mmol fonction méthylcarboxylique acide) sont solubilisés dans le DMF à 60 g/L puis refroidis à 0°C. 1,32 g de glycinate de L-menthol, sel d'acide chlorhydrique (5,29 mmol) est mis en suspension dans du DMF à 100 g/L. 0,54 g de triéthylamine (5,29 mmol) est ensuite ajouté à cette suspension. Une fois la solution de polymère à 0°C, une solution de NMM (6,59 g, 65,1 mmol) dans le DMF (530 g/L) et 7,07 g (65,1 mmol) de EtOCOCl sont ensuite ajoutés. Après 10 minutes de réaction, la suspension de glycinate de L-menthol est ajoutée. Le milieu est ensuite maintenu à 10°C pendant 45 minutes. Le milieu est ensuite chauffé à 50°C. Une solution d'imidazole (14,7 g dans 22 mL d'eau) et 65 mL d'eau sont ajoutés dans le milieu réactionnel. La solution de polymère est ultrafiltrée sur membrane PES 10 kD contre 6 volumes de solution NaCl 0,9 %, 4 volumes de soude 0,01N, 7 volumes de solution NaCl 0,9 % puis 3 volumes d'eau. La concentration de la solution de polymère est déterminée par extrait sec. Une fraction de solution est lyophilisée et analysée par RMN ¹H dans D₂O pour déterminer le taux de fonctions acides converties en amide de glycinate de L-menthol.

D'après l'extrait sec : [polymère modifié] = 25,7 mg/g

D'après la RMN ¹H : la fraction molaire des acides modifiés par le glycinate de L-menthol par unité saccharidique est de 0,09.

### Exemple 7 : Synthèse de dextraneméthylcarboxylate de sodium modifié par l'alaninate de (±)α-tocophérol

L'alaninate de (±)α-tocophérol, sel d'acide chlorhydrique est obtenu selon le procédé décrit dans J. Pharm. Sci. 1995, 84(1), 96-100.

Par un procédé similaire à celui décrit à l'exemple 6, un dextraneméthylcarboxylate de sodium modifié par l'alaninate de (±)o-tocophérol est obtenu.

D'après l'extrait sec : [polymère modifié] = 28,1 mg/g

D'après la RMN ¹H : la fraction molaire des acides modifiés par l'alaninate de (±)α-tocophérol par unité saccharidique est de 0,04.

### Exemple 8: Synthèse de dextraneméthylcarboxylate de sodium modifié par le glycinate d'octanol

Le glycinate d'octanol, sel d'acide paratoluènesulfonique est obtenu selon le procédé décrit dans le brevet (Kenji, M et al., US4826818).

Par un procédé similaire à celui décrit à l'exemple 6, un dextraneméthylcarboxylate de sodium modifié par le glycinate d'octanol est obtenu.

D'après l'extrait sec : [polymère modifié] = 34,1 mg/g

D'après la RMN ¹H : la fraction molaire des acides modifiés par le glycinate d'octanol par unité saccharidique est de 0,27.

### Exemple 9: Synthèse de dextraneméthylcarboxylate de sodium modifié par le phénylalaninate d'octanol

Le phénylalaninate d'octanol, sel d'acide paratoluènesulfonique est obtenu selon le procédé décrit dans le brevet (Kenji, M et al., US4826818).

Par un procédé similaire à celui décrit à l'exemple 6, un dextraneméthylcarboxylate de sodium modifié par le phénylalaninate d'octanol est obtenu.

D'après l'extrait sec : [polymère modifié] = 30,2 mg/g

D'après la RMN ¹H : la fraction molaire des acides modifiés par le phénylalaninate d'octanol par unité saccharidique est de 0,09.

### Exemple 10: Synthèse de dextraneméthylcarboxylate de sodium modifié par le phénylalaninate d'alcool benzylique

Par un procédé similaire à celui décrit à l'exemple 6, un dextraneméthylcarboxylate de sodium modifié par le phénylalaninate d'alcool benzylique est obtenu en utilisant le phénylalaninate d'alcool benzylique, sel d'acide chlorhydrique (Bachem).

D'après l'extrait sec : [polymère modifié] = 47,7 mg/g

D'après la RMN ¹H : la fraction molaire des acides modifiés par le phénylalaninate d'alcool benzylique par unité saccharidique est de 0,41.

### Exemple 11 : Synthèse de dextraneméthylcarboxylate de sodium modifié par le phénylalaninate d'isohexanol.

Le phénylalaninate d'isohexanol, sel d'acide paratoluène sulfonique est obtenu selon le procédé décrit dans le brevet (Kenji, M et al., US4826818).

Par un procédé similaire à celui décrit à l'exemple 6, un dextraneméthylcarboxylate de sodium modifié par le phénylalaninate d'isohexanol est obtenu.

D'après l'extrait sec : [polymère modifié] = 29,8 mg/g

D'après la RMN ¹H : la fraction molaire des acides modifiés par le phénylalaninate d'isohexanol par unité saccharidique est de 0,18.

### Exemple 12 : Solubilisation d'un lyophilisat de BMP-2.

Un essai de solubilisation d'un lyophilisat de Bone Morphogenetic Protein 2 (BMP-2) a été développé afin de mettre en évidence le pouvoir solubilisant de différents polymères à pH physiologique. La BMP-2 est solubilisée dans un tampon contenant du sucrose (Sigma), de la glycine (Sigma), de l'acide glutamique (Sigma), du chlorure de sodium (Riedel-de-Haën), du polysorbate 80 (Fluka). Le pH de cette solution est ajusté à pH 4,5 par ajout de soude puis est la solution lyophilisée. 283,2 mg de lyophilisat contiennent environ 12 mg de BMP-2.

Les polymères selon l'invention sont mis en oeuvre dans ce test. A titre comparatif, un polymère décrit dans la demande de brevet FR0702316 est également mis en oeuvre dans ce test, le dextraneméthylcarboxylate de sodium modifié par le phénylalaninate d'éthyle.

Le test consiste à introduire environ exactement 4 mg de lyophilisat contenant 0,168 mg de BMP-2. Le lyophilisat est ensuite repris par 210 µL d'une solution aqueuse pour atteindre une concentration finale en BMP-2 de 0,8 mg/mL à pH physiologique, la concentration finale de polymère étant de 5 mg/ml.

L'aspect visuel de la solution est noté après 5 minutes d'agitation à vitesse réduite sur rouleau.

Les résultats pour différentes solutions sont rassemblés dans le tableau suivant.

| Solution | Aspect visuel | pH |
|---|---|---|
| Eau | limpide | 4.3 |
| Exemple 9 | limpide | 7.4 |
| Exemple 8 | limpide | 7.5 |
| Exemple 5 | limpide | 7.4 |
| Contre-exemple FR0702316 | trouble | 7.5 |

L'ajout d'eau conduit à une solution limpide de BMP-2 mais à pH acide.

Ce test permet de mettre en évidence l'amélioration de la solubilisation de la BMP-2 à pH physiologique par les polymères selon l'invention. En revanche, le dextraneméthylcarboxylate de sodium modifié par le phénylalaninate d'éthyle ne permet pas d'obtenir une solution limpide de BMP-2.

## Revendications

1. Polysaccharide comportant des groupes fonctionnels carboxyles dont un au moins est substitué par un dérivé d'alcool hydrophobe, noté Ah :
• ledit alcool (Ah) étant greffé ou lié au polysaccharide anionique par un bras de couplage R, ledit bras de couplage étant lié au polysaccharide anionique par une fonction F ladite fonction F résultant du couplage entre la fonction amine du bras de liaison R et une fonction carboxyle du polysaccharide anionique, et ledit bras de couplage étant lié à l'alcool hydrophobe par une fonction G résultant du couplage entre une fonction carboxyle, isocyanate, thioacide ou alcool du bras de couplage et une fonction de l'alcool hydrophobe, les fonctions carboxyles du polysaccharide anionique non substituées étant sous forme de carboxylate de cation.
- F étant une fonction amide,
- G étant soit une fonction ester, thioester, carbonate, carbamate,
- R étant une chaîne comprenant entre 1 et 18 carbones, éventuellement branchée et/ou insaturée, éventuellement comprenant un ou plusieurs hétéroatomes, et ayant au moins une fonction acide,
• Ah étant un reste d'un alcool hydrophobe, produit du couplage entre la fonction hydroxyle de l'alcool hydrophobe et au moins une fonction électrophile portée par le groupement R.
ledit polysaccharide comportant des groupes fonctionnels carboxyles étant amphiphile à pH neutre.

2. Polysaccharide selon la revendication 1, **caractérisé en ce qu'**il est choisi parmi les polysaccharides comportant des groupes fonctionnels carboxyles dont un au moins est substitué par un dérivé d'alcool hydrophobe, de formule générale I : 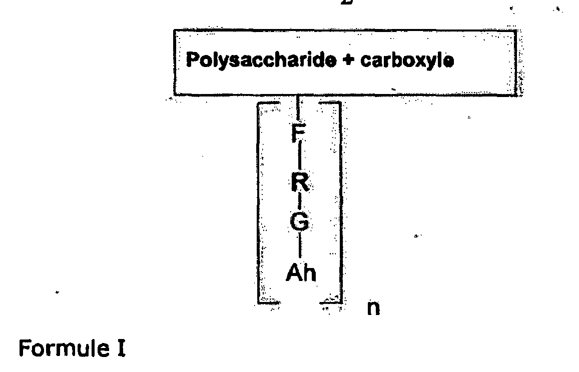
- dans laquelle, n représente la fraction molaire des fonctions carboxyles du polysaccharide substituées par F-R-G-Ah et est compris entre 0,01 et 0,7,
- F, R, G et Ah répondant aux définitions données ci-dessus, et lorsqu'une ou des fonctions carboxyles du polysaccharide ne sont pas substituée par F-R-G-Ah, alors la ou les fonctions carboxyles du polysaccharide sont des carboxylates de cation.

3. Polysaccharide selon l'une quelconque des revendications précédentes, **caractérisé en ce que** le polysaccharide anionique est naturellement porteur de fonctions acides et est choisi dans le groupe constitué par l'alginate, le hyaluronane, le galacturonane.

4. Polysaccharide selon l'une quelconque des revendications précédentes, **caractérisé en ce que** le polysaccharide anionique est un polysaccharide anionique synthétique obtenu à partir d'un polysaccharide naturel anionique ou non anionique (neutre) sur lequel au moins une fonction acide a été greffée de formule générale II. 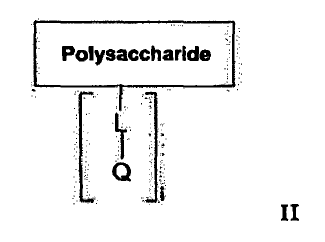
- le polysaccharide naturel étant choisi dans le groupe des polysaccharides constitués en majorité de monomères glycosidiques liés par des liaisons glycosidiques de type (1,6) et/ou (1,4) et/ou (1,3) et/ou (1,2),
- L étant une liaison résultant du couplage entre le bras de liaison Q et une fonction -OH du polysaccharide neutre ou anionique, étant soit une fonction ester, thionoester, carbonate, carbamate ou éther,
- Q étant une chaîne comprenant entre 1 et 18 carbones, éventuellement branchée et/ou insaturée comprenant un ou plusieurs hétéroatomes, et ayant au moins une fonction acide, CO₂H.

5. Polysaccharide selon la revendication 4, **caractérisé en ce que** le polysaccharide est constitué en majorité de monomères glycosidiques liés par des liaisons glycosidiques de type (1,6).

6. Polysaccharide selon la revendication 5, **caractérisé en ce que** le polysaccharide est constitué en majorité de monomères glycosidiques liés par des liaisons glycosidiques de type (1,6) est le dextrane.

7. Polysaccharide selon la revendication 4, **caractérisé en ce que** le polysaccharide est constitué en majorité de monomères glycosidiques liés par des liaisons glycosidiques de type (1,4).

8. Polysaccharide selon la revendication 7, **caractérisé en ce que** le polysaccharide constitué en majorité de monomères glycosidiques liés par des liaisons glycosidiques de type (1,4) est choisi dans le groupe constitué par le pullulane, l'alginate, le hyaluronane, le xylane, le galacturonane ou une cellulose soluble dans l'eau.

9. Polysaccharide selon l'une quelconque des revendications 4 à 8, **caractérisé en ce que** le groupe Q est choisi dans les groupes suivants :

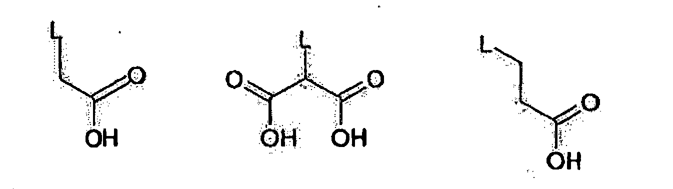

10. Polysaccharide selon l'une quelconque des revendications 1 à 9, **caractérisé en ce que** le groupement R est choisi parmi les acides aminés.

11. Polysaccharide selon l'une quelconque des revendications 1 à 10, **caractérisé en ce que** le groupement R est choisi parmi les alpha acides aminés.

12. Polysaccharide selon la revendication 11, **caractérisé en ce que** les alpha acides aminés sont choisis parmi les alpha acides aminés naturels.

13. Polysaccharide selon l'une quelconque des revendications 1 à 12, **caractérisé en ce que** l'alcool hydrophobe est choisi parmi les alcools gras.

14. Polysaccharide selon la revendication 13, **caractérisé en ce que** l'alcool hydrophobe est choisi parmi les alcools constitués d'une chaîne alkyle insaturée ou saturée comprenant de 4 à 18 carbones.

15. Polysaccharide selon l'une quelconque des revendications 1 à 13, **caractérisé en ce que** l'alcool gras est choisi parmi le méristyl, le cétyl, le stéaryl, le cétéaryl, le butyl, l'oléyl, la lanoline.

16. Polysaccharide selon la revendication 13, **caractérisé en ce que** l'alcool hydrophobe est le cholestérol.

17. Polysaccharide selon la revendication 13, **caractérisé en ce que** l'alcool hydrophobe est le menthol.

18. Polysaccharide selon la revendication 13, **caractérisé en ce que** l'alcool hydrophobe est choisi parmi les tocophérols.

19. Polysaccharide selon la revendication 13, **caractérisé en ce que** l'alcool hydrophobe est choisi parmi les alcools porteurs de groupe aryle.

20. Utilisation d'un polysaccharide fonctionnalisé selon l'une quelconque des revendications précédentes pour la préparation de compositions pharmaceutiques.

21. Composition pharmaceutique comprenant un polysaccharide selon l'une quelconque des revendications 1 à 19 et au moins un principe actif.

22. Composition pharmaceutique selon la revendication 21, **caractérisée en ce que** le principe actif est choisi dans le groupe constitué par les protéines, les glycoprotéines, les peptides et les molécules thérapeutiques non-peptidiques.

## Patentansprüche

1. Polysaccharid umfassend funktionelle Carboxygruppen, von denen wenigstens eine durch ein Derivat eines hydrophoben Alkohols, bezeichnet als HA, substituiert ist, wobei:
- der Alkohol (HA) aufgepropft ist auf oder mit dem anionischen Polysaccharid verbunden ist durch einen Kupplungsarm R, der Kupplungsarm mit dem anionischen Polysaccharid durch eine Funktion F verbunden ist, die Funktion F sich aus der Kupplung zwischen der Amin-Funktion des Kupplungsarms R und einer Carboxyl-Funktion des anionischen Polysaccharids ergibt, und der Kupplungsarm mit dem hydrophoben Alkohol verbunden ist durch eine Funktion G, die sich aus der Kupplung zwischen einer Carboxyl-, Isocyanat-, Thiosäure-Funktion oder Alkohol-Funktion des Kupplungsarmes und einer Funktion des hydrophoben Alkohols ergibt, die nicht-substituierten Carboxyl-Funktionen des anionischen Polysaccharids als kationisches Carboxylat vorliegen;
- F eine Amid-Funktion ist;
- G ein Ester-, Thioester-, Carbonat- oder Carbamat-Funktion ist,
- R eine Kette ist umfassend zwischen 1 und 18 Kohlenstoffen, die optional verzweigt und/oder ungesättigt ist, optional ein oder mehrere Heteroatome umfasst, und wenigstens eine Säure-Funktion aufweist,
- HA ein Rest eines hydrophoben Alkohols ist, hergestellt durch die Kupplung zwischen der Hydroxyl-Funktion des hydrophoben Alkohols und wenigstens einer elektrophilen, von der Gruppe R getragenen Funktion,
das funktionelle Carboxylgruppen umfassende Polysaccharid bei neutralem pH amphiphil ist.

2. Polysaccharid nach Anspruch 1, **dadurch gekennzeichnet, dass** es ausgewählt ist aus den Polysacchariden umfassend funktionelle Carboxylgruppen, von denen wenigstens eine durch ein Derivat eines hydrophoben Alkohol substituiert ist, der allgemeinen Formel I 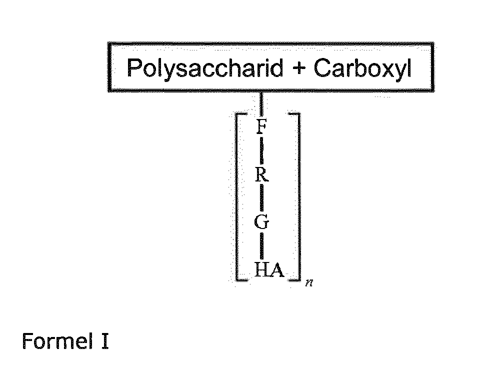
- in der n den molaren Anteil der Carboxyl-Funktionen des Polysaccharids darstellt, die durch F-R-G-HA substituiert sind, und zwischen 0,01 und 0,7 beträgt,
- F, R, G und HA den obigen Definition entsprechen und wenn eine oder mehrere der Carboxyl-Funktionen des Polysaccharids nicht durch F-R-G-HA substituiert ist/sind, dann ist/sind die Carboxyl-Funktion(en) des Polysaccharids kationische(s) Carboxylat(e).

3. Polysaccharid nach einem der vorangehenden Ansprüche, **dadurch gekennzeichnet, dass** das anionische Polysaccharid natürlicherweise Säure-Funktionen trägt und aus der Gruppe bestehend aus Alginat, Hyaluronan und Galakturonan ausgewählt ist.

4. Polysaccharid nach einem der vorangehenden Ansprüche, **dadurch gekennzeichnet, dass** das anionische Polysaccharid ein synthetisches anionisches Polysaccharid ist, das ausgehend von einem natürlichen anionischen oder einem nichtanionischen (neutralen) Polysaccharid erhalten ist, auf das wenigstens eine Säure-Funktion aufgepfropft ist, der allgemeinen Formel (II) 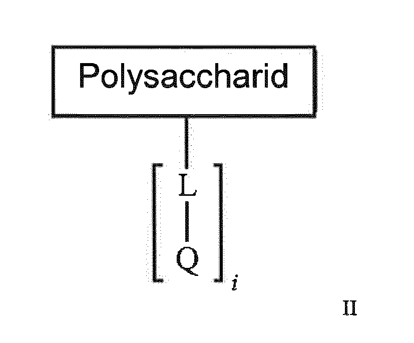 wobei
- das natürliche Polysaccharid aus der Gruppe der Polysaccharide ausgewählt ist, die hauptsächlich aus glykosidischen Monomeren gebildet sind, die durch glykosidische Bindungen vom Typ (1,6) und/oder (1,4) und/oder (1,3) und/oder (1,2) verbunden sind,
- L eine Bindung ist, die entsteht durch die Kupplung zwischen dem Verbindungsarm Q und einer Funktion -OH des neutralen oder anionischen Polysaccharids, welche eine Ester-, Thionoester-, Carbonat-, Carbamat- oder Ether-Funktion ist,
- Q eine Kette ist, die 1 bis 18 Kohlenstoffe umfasst, optional verzweigt und/oder ungesättigt, und ein oder mehrere Heteroatome umfasst, und wenigstens eine Säure-Funktion CO₂H aufweist.

5. Polysaccharid nach Anspruch 4, **dadurch gekennzeichnet, dass** das Polysaccharid hauptsächlich aus glykosidischen Monomeren besteht, die durch glykosidische Bindungen vom Typ (1,6) verbunden sind.

6. Polysaccharid nach Anspruch 5, **dadurch gekennzeichnet, dass** das Polysaccharid, das hauptsächlich aus glykosidischen Monomeren besteht, die durch glykosidische Bindungen vom Typ (1,6) verbunden sind, Dextran ist.

7. Polysaccharid nach Anspruch 4, **dadurch gekennzeichnet, dass** das Polysaccharid hauptsächlich aus glykosidischen Monomeren besteht, die durch glykosidische Bindungen vom Typ (1,4) verbunden sind.

8. Polysaccharid nach Anspruch 7, **dadurch gekennzeichnet, dass** das Polysaccharid, das hauptsächlich aus glykosidischen Monomeren besteht, die durch glykosidische Bindungen vom Typ (1,4) verbunden sind, ausgewählt ist aus der Gruppe bestehend aus Pullulan, Alginat, Hyaluronan, Xylan, Galacturonan und einer wasserlöslichen Cellulose.

9. Polysaccharid nach einem der Ansprüche 4 bis 8, **dadurch gekennzeichnet, dass** die Gruppe Q aus den folgenden Gruppen ausgewählt ist

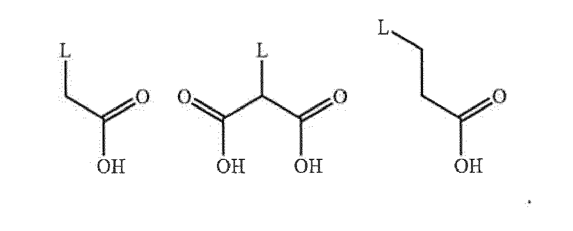

10. Polysaccharid nach einem der Ansprüche 1 bis 9, **dadurch gekennzeichnet, dass** die Gruppe R aus der Gruppe der Aminosäuren ausgewählt ist.

11. Polysaccharid nach einem der Ansprüche 1 bis 10, **dadurch gekennzeichnet, dass** die Gruppe R aus der Gruppe der alpha-Aminosäuren ausgewählt ist.

12. Polysaccharid nach Anspruch 11, **dadurch gekennzeichnet, dass** die alpha-Aminosäuren aus den natürlichen Aminosäuren ausgewählt sind.

13. Polysaccharid nach einem der Ansprüche 1 bis 12, **dadurch gekennzeichnet, dass** der hydrophobe Alkohol aus der Gruppe der fetten Alkohole ausgewählt ist.

14. Polysaccharid nach Anspruch 13, **dadurch gekennzeichnet, dass** der hydrophobe Alkohol aus der Gruppe der Alkohole mit einer ungesättigten oder einer gesättigten Alkylkette, umfassend 4 bis 18 Kohlenstoffe, ausgewählt ist.

15. Polysaccharid nach einem der Ansprüche 1 bis 13, **dadurch gekennzeichnet, dass** der fette Alkohol aus der Gruppe bestehend aus Myristylalkohol, Cetylalkohol, Stearylalkohol, Cetearylalkohol, Butylalkohol, Oleylalkohol und Lanolin ausgewählt ist.

16. Polysaccharid nach Anspruch 13, **dadurch gekennzeichnet, dass** der hydrophobe Alkohol Cholesterol ist.

17. Polysaccharid nach Anspruch 13, **dadurch gekennzeichnet, dass** der hydrophobe Alkohol Menthol ist.

18. Polysaccharid nach Anspruch 13, **dadurch gekennzeichnet, dass** der hydrophobe Alkohol aus der Gruppe der Tocopherole ausgewählt ist.

19. Polysaccharid nach Anspruch 13, **dadurch gekennzeichnet, dass** der hydrophobe Alkohol aus der Gruppe der Alkohole, die eine Arylgruppe tragen, ausgewählt ist.

20. Verwendung eines funktionalisierten Polysaccharids nach einem der vorangehenden Ansprüche für die Herstellung pharmazeutischer Zusammensetzungen.

21. Pharmazeutische Zusammensetzung umfassend ein Polysaccharid nach einem der Ansprüche 1 bis 19 und wenigstens ein aktives Prinzip.

22. Pharmazeutische Zusammensetzung nach Anspruch 21, **dadurch gekennzeichnet, dass** das aktive Prinzip aus der Gruppe bestehend aus Proteinen, Glycoproteinen, Peptiden und nicht-peptidischen therapeutischen Molekülen, ausgewählt ist.

## Claims

1. Polysaccharide comprising functional carboxyl groups, of which at least one is substituted by a hydrophobic alcohol derivative, denoted Ha:
• said alcohol (Ha) being grafted or linked to the anionic polysaccharide by a coupling arm R, said coupling arm being linked to the anionic polysaccharide by a functional group F, the said functional group F resulting from the coupling, between the amine functional group of the connecting arm R and a functional carboxyl group of the anionic polysaccharide, and said coupling arm being linked to the hydrophobic alcohol by a functional group G resulting from the coupling between a carboxyl, isocyanate, thioacid or alcohol functional group of the coupling arm and a functional group of the hydrophobic alcohol, the unsubstituted functional carboxyl groups of the anionic polysaccharide being in the cation carboxylate form,
- F being a functional amide group,
- G being a functional ester, thioester, carbonate or carbamate group,
- R being a chain comprising between 1 and 18 carbons which is optionally branched and/or unsaturated, which optionally comprises one or more heteroatoms and which has at least one functional acid group,
• Ha being a residue of a hydrophobic alcohol, the product of the coupling between the functional hydroxyl group of the hydrophobic alcohol and at least one functional electrophilic group carried by the R group,
said polysaccharide comprising functional carboxyl groups being amphiphilic at neutral pH.

2. Polysaccharide according to Claim 1, **characterized in that** it is chosen from the polysaccharides comprising functional carboxyl groups, of which at least one is substituted by a hydrophobic alcohol derivative of general formula I: 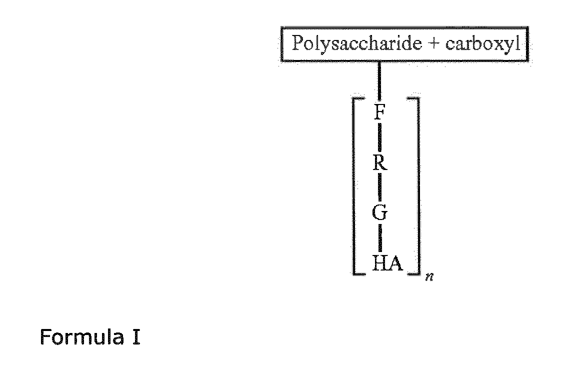
- in which n represents the molar fraction of the functional carboxyl groups of the polysaccharide substituted by F-R-G-Ha and is between 0.01 and 0.7,
- F, R, G and Ha corresponding to the definitions given above and, when one or more functional carboxyl groups of the polysaccharide are not substituted by F-R-G-Ha, then the functional carboxyl group or groups of the polysaccharide are cation carboxylates.

3. Polysaccharide according to either one of the preceding claims, **characterized in that** the anionic polysaccharide naturally carries functional acid groups and is chosen from the group consisting of alginate, hyaluronan and galacturonan.

4. Polysaccharide according to any one of the preceding claims, **characterized in that** the anionic polysaccharide is a synthetic anionic polysaccharide obtained from a natural anionic or nonanionic (neutral) polysaccharide to which at least one functional acid group has been grafted, of general formula II: 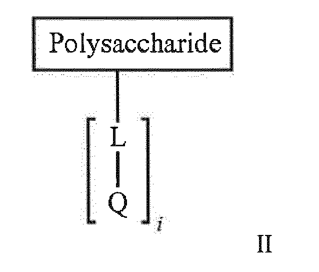 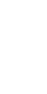
- the natural polysaccharide being chosen from the group of polysaccharides predominantly composed of glycoside monomers linked via (1,6) and/or (1,4) and/or (1,3) and/or (1,2) glycoside bonds,
- L being a bond resulting from the coupling between the connecting arm Q and a functional -OH group of the neutral or anionic polysaccharide, being either an ester, thionoester, carbonate, carbamate or functional ether group,
- Q being a chain comprising between 1 and 18 carbons which is optionally branched and/or unsaturated, which comprises one or more heteroatoms and which has at least one CO₂H functional acid group.

5. Polysaccharide according to Claim 4, **characterized in that** the polysaccharide is predominantly composed of glycoside monomers linked via (1,6) glycoside bonds.

6. Polysaccharide according to Claim 5, **characterized in that** the polysaccharide predominantly composed of glycoside monomers linked via (1,6) glycoside bonds is dextran.

7. Polysaccharide according to Claim 4, **characterized in that** the polysaccharide is predominantly composed of glycoside monomers linked via (1,4) glycoside bonds.

8. Polysaccharide according to Claim 7, **characterized in that** the polysaccharide predominantly composed of glycoside monomers linked via (1,4) glycoside bonds is chosen from the group consisting of pullulan, alginate, hyaluronan, xylan, galacturonan or a water-soluble cellulose.

9. Polysaccharide according to any one of Claims 4 to 8, **characterized in that** the Q group is chosen from the following groups:

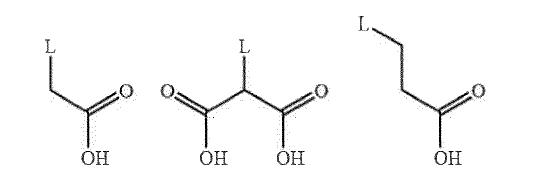

10. Polysaccharide according to any one of Claims 1 to 9, **characterized in that** the R group is chosen from amino acids.

11. Polysaccharide according to any one of Claims 1 to 10, **characterized in that** the R group is chosen from α-amino acids.

12. Polysaccharide according to Claim 11, **characterized in that** the α-amino acids are chosen from natural α-amino acids.

13. Polysaccharide according to any one of Claims 1 to 12, **characterized in that** the hydrophobic alcohol is chosen from fatty alcohols.

14. Polysaccharide according to Claim 13, **characterized in that** the hydrophobic alcohol is chosen from the alcohols composed of an unsaturated or saturated alkyl chain comprising from 4 to 18 carbons.

15. Polysaccharide according to any one of Claims 1 to 13, **characterized in that** the fatty alcohol is chosen from myristyl alcohol, cetyl alcohol, stearyl alcohol, cetearyl alcohol, butyl alcohol, oleyl alcohol or lanolin.

16. Polysaccharide according to Claim 13, **characterized in that** the hydrophobic alcohol is cholesterol.

17. Polysaccharide according to Claim 13, **characterized in that** the hydrophobic alcohol is menthol.

18. Polysaccharide according to Claim 13, **characterized in that** the hydrophobic alcohol is chosen from tocopherols.

19. Polysaccharide according to Claim 13, **characterized in that** the hydrophobic alcohol is chosen from alcohols carrying an aryl group.

20. Use of a functionalized polysaccharide according to any one of the preceding claims in the preparation of pharmaceutical compositions.

21. Pharmaceutical composition comprising a polysaccharide according to any one of Claims 1 to 19 and at least one active principle.

22. Pharmaceutical composition according to Claim 21, **characterized in that** the active principle is chosen from the group consisting of proteins, glycoproteins, peptides and non-peptide ther
